# EUROPEAN PATENT APPLICATION

(11) **EP 4 659 752 A1**
(43) Date of publication of application: **10.12.2025**
(21) Application number: 24750454.1
(22) Date of filing: 16.01.2024
(51) Int. Cl.: A61K 31/7088, A61P 27/02, C12N 15/113

(54) **PHARMACEUTICAL COMPOSITION USING ANTISENSE OLIGONUCLEOTIDE (ASO) FOR PREVENTION OR TREATMENT AVELLINO CORNEAL DYSTROPHY**

(30) Priority: 01.02.2023 KR 20230013666; 21.08.2023 KR 20230108737
(71) Applicant: Medicibio Co., Ltd, Yongin-si, Gyeonggi-do 17095 (KR)
(72) Inventor: KI, Min Hyo, Yongin-si Gyeonggi-do 17095 (KR); CHOI, Mee-Hwa, Yongin-si Gyeonggi-do 16874 (KR); PARK, So Hyun, Yongin-si Gyeonggi-do 17011 (KR); LEE, Kug Hwa, Yongin-si Gyeonggi-do 17095 (KR)
(74) Representative: Mewburn Ellis LLP
(86) International application number: PCT/KR2024/000734
(87) International publication number: WO 2024/162652

(57) **Abstract**

The present invention relates to: a pharmaceutical composition comprising an antisense oligonucleotide that inhibits the expression of R124H mutant of TGFBI protein for the prevention or treatment of Avellino corneal dystrophy; uses of the composition; or a treatment method thereof. In particular, the present invention selectively targets R124H TGFBI mRNA, a precursor of the R124H TGFBI protein that causes Avellino corneal dystrophy, thereby inhibiting R124H TGFBI expression while minimizing the effect on normal WT TGFBI expression, and thus can effectively prevent or treat Avellino corneal dystrophy.

## Description

### Technical Field

The present disclosure relates to an ASO pharmaceutical composition that inhibits expression of R124H mutant TGFBI, a pathogenic protein, for the purpose of preventing or treating Avellino corneal dystrophy, a use thereof, and a treatment method thereof.

### Background Art

The cornea is an important tissue of the eye to secure the field of vision and maintain eyesight. It is essential to keep the cornea clear since the cloudy cornea directly leads to reduced vision and inconvenience in daily life. A disease in which the cornea becomes cloudy and vision deteriorates due to the formation of opaque deposits on the cornea is called corneal dystrophy. Symptoms of this condition often worsen gradually with age.

Among corneal dystrophies, granular corneal dystrophy is a slowly progressive corneal abnormality that usually begins in infancy and gradually makes the cornea cloudy, resulting in severe vision loss after the age of 60. It is also referred to as granular corneal degeneration, granular corneal dystrophy, or granular type corneal dystrophy. There are two types of granular corneal dystrophy. The granular corneal dystrophy type 1, a very rare form, was first described by Arthur Groenouw in Germany in 1890. The granular corneal dystrophy type 2 (Avelino corneal dystrophy), also a very rare form of corneal dystrophy, was first described by Folberg in 1988 and named after the first four patients, who originated from the Avellino region of Italy.

It is known that the granular corneal dystrophy is caused by point mutation in the transforming growth factor β-induced (TGFBI) gene located on chromosome 5q31. Among these, the R124H mutation (R124H TGFBI), in which arginine at position 124 of the TGFBI protein is substituted with histidine, is one of the most frequently observed mutations and causes granular corneal dystrophy type 2, also known as Avellino dystrophy.

TGFBI, a 68 kDa extracellular matrix protein consisting of 683 amino acids, is mainly involved in cell adhesion, migration, and differentiation. It is primarily expressed in corneal fibroblast (keratocyte) and corneal epithelial cells, where it interacts with the collagen components of the corneal stroma and plays a role in corneal wound healing and construction of extra cellular matrix. However, unlike the normal protein, mutant forms of TGFBI do not fold properly in three dimensions. As a result, they aggregate very easily and are resistant to degradation, leading to the accumulation of opaque, insoluble deposits in the corneal stroma or Bowman's membrane, thus causing granular corneal dystrophy.

Granular corneal dystrophy type 2 (GCD 2), also known as Avellino corneal dystrophy, is an autosomal dominant genetic disease, and approximately 1 in 870 people in Korea carry the R124H TGFBI mutation. Most patients are heterozygous for the mutation, with symptom prevalence and severity increasing with age. Homozygous mutants are extremely rare (approximately 1/1,000 or 1/10,000 compared to heterozygous mutants), and typically develop symptoms during childhood that are more severe than those of heterozygous individuals.

Current treatments include corneal transplantation and superficial keratectomy to remove the area of the cornea where the deposits have formed. In the case of corneal transplantation, it is possible to maintain a cloud-free state for a long period but is a highly invasive procedure, requiring a donor and carrying the risk of recurrence. In the case of keratectomy, it is a less invasive and helps maintain vision temporarily before corneal transplantation, but requires repeated procedures to maintain the cornea transparent. This repeated treatment often causes presbyopia due to a decrease in refractive index, along with a high risk of recurrence and deterioration due to genetic predisposition.

Various research efforts have aimed to develop therapeutic drugs, but no successful treatments have emerged to date. For example, KR 10-1370659 aimed to reduce protein deposits through autophagy induced by rapamycin or melatonin, and KR 10-1394538 attempted to reduce TGFBI protein expression using vitamin D3. US 2008/0267946 attempted using TGF-β antibodies to alleviate symptoms aggravated by TGF-β activation through strong light exposure, such as ultraviolet light in patients with Avellino corneal dystrophy.

However, these attempts have not led to successful drug development to date, and the mechanism itself has limitations in that it is not directly related to the pathogenic R124H TGFBI. In particular, the R124H TGFBI protein, once aggregated in the cornea, is very difficult to dissolve or remove with drugs. Even the surgery may trigger new aggregation. Therefore, a more effective strategy would be to prevent the aggregation of the pathogenic protein at an earlier stage by specifically inhibiting R124H TGFBI.

Antisense oligonucleotides (ASOs) are single-stranded DNA-based oligonucleotides that specifically bind to mRNA with a complementary sequence to suppress expression of target proteins by recruiting an RNase called RNase H, which degrades the mRNA, or by blocking translation into protein. The present disclosure aims to propose a method of fundamentally treating or preventing Avellino corneal dystrophy induced by R124H TGFBI using an ASO that specifically inhibits expression of R124H TGFBI, the pathogenic p of Avellino corneal dystrophy.

### Disclosure of the Invention

### Technical Goals

An object of the present disclosure is to provide a pharmaceutical composition for preventing or treating Avellino corneal dystrophy, which inhibits the expression of R124H TGFBI using ASO, a use thereof, and a treatment method thereof.

In addition, another object of the present disclosure is to provide a pharmaceutical composition for preventing or treating Avellino corneal dystrophy, which selectively inhibits R124H mutant TGFBI while minimizing effects on normal TGFBI (wild-type TGFBI) expression using ASO, a use thereof, and a treatment method thereof.

### Technical Solutions

To achieve the above object, the present disclosure provides a pharmaceutical composition for preventing or treating Avellino corneal dystrophy, comprising an antisense oligonucleotide having a nucleotide sequence that inhibits R124H mutant TGFBI expression.

In addition, the present disclosure provides the use of an antisense oligonucleotide having a nucleotide sequence that inhibits the expression of TGFBI with the R124H mutation, as an agent for prevention or treatment of Avellino corneal dystrophy.

In addition, the present disclosure provides a method for preventing or treating Avellino corneal dystrophy, the method including administering an antisense oligonucleotide having a nucleotide sequence that inhibits the expression of R124H mutant TGFBI. Advantageous Effects

Development of a gene therapy is necessary for effective treatment of Avellino corneal dystrophy, a genetic disease with very high medical unmet needs. In particular, once the pathogenic R124H TGFBI protein aggregates in the cornea, it is difficult to dissolve or remove it with drugs, and even surgery may trigger new aggregation. Therefore, in order to block the pathogenic mechanism, the present disclosure provides a technical advantage by using R124H TGFBI-specific antisense oligonucleotides to act at the mRNA stage, prior to the production of the R124H TGFBI protein. By inhibiting the expression of R124H TGFBI, the causative protein of Avellino corneal dystrophy, the disease can be effectively prevented or treated.

### Brief Description of Drawings

FIG. 1 shows diagrams displaying coding mRNA sequences and the corresponding amino acid sequences at a position including amino acid 124 of a normal WT TGFBI protein and a mutant R124H TGFBI protein.

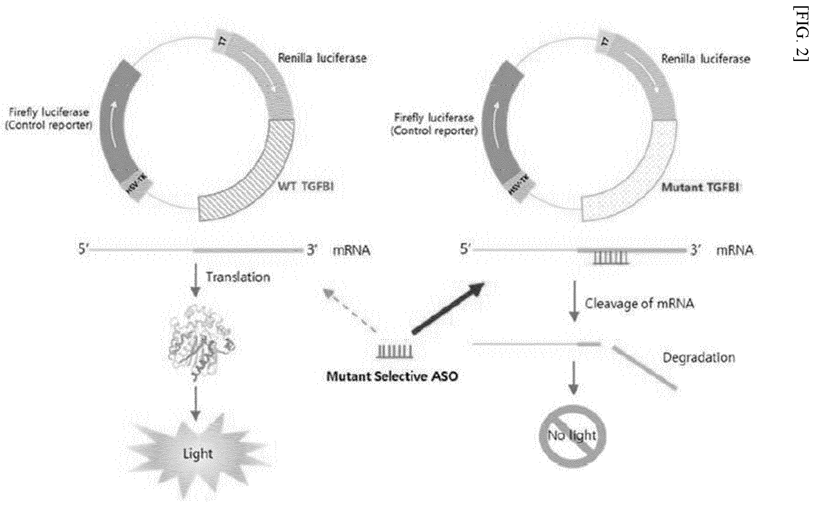

FIG. 2 shows a diagram of a test method for deriving ASOs that selectively inhibit R124H TGFBI, using a reporter gene system that indicates an expression level of WT TGFBI protein or that of R124H TGFBI protein.
FIG. 3 shows results of detecting an expression level of WT TGFBI protein and that of R124H TGFBI protein after transfection with ASO at indicated concentrations.
FIG. 4 shows a result of detecting an expression level of WT TGFBI protein and that of R124H TGFBI protein after treatment (gymnotic delivery) with ASO without a transfection reagent.
FIG. 5 shows results of detecting an expression level of WT TGFBI protein and that of R124H TGFBI protein after treatment (gymnotic delivery) with ASO at various concentrations without a transfection reagent.

### Best Mode for Carrying Out the Invention

Hereinafter, preferred embodiments of the present disclosure will be described. However, the embodiments of the present disclosure may be modified into various other forms, and the scope of the present disclosure is not limited to the embodiments described below.

The present disclosure aims to prevent or treat Avellino corneal dystrophy by inhibiting production of TGFBI protein, the causative protein of the disease, using DNA-based antisense oligonucleotides (ASOs), and more preferably, to prevent or treat Avellino corneal dystrophy by selectively inhibiting the production of R124H mutant TGFBI proteins among TGFBI proteins.

Avellino corneal dystrophy (granular corneal dystrophy type 2) to be treated herein is a dominant genetic disease caused by an R124H mutation in the TGFBI protein. The resulting mutant TGFBI protein is misfolded and unstable, leading to the formation of granular deposits in the stromal layer of the cornea, thereby gradually reducing corneal transparency and eventually causing loss of vision.

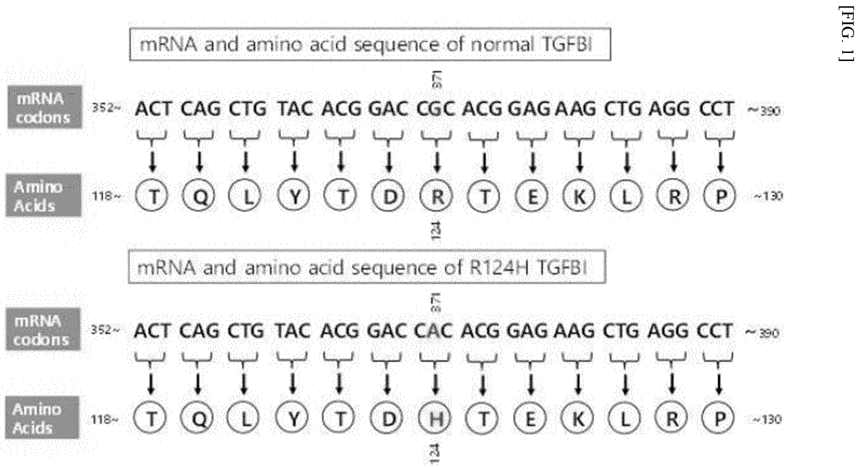

The transforming growth factor β-induced (TGFBI) protein associated with Avellino corneal dystrophy is produced from an mRNA which has 2,052 unit nucleotides and 684 codons and is ultimately translated into a protein composed of 683 amino acids. Avellino corneal dystrophy is caused by a point mutation from CGC to CAC induced in the 124^{th} codon in a TGFBI coding sequence (CDS) (FIG. 1), and it is a serious genetic disease for which there is currently no treatment and which easily relapses even with surgery, thereby gradually leading to vision loss.

In addition, depending on the allele, the majority of patients with Avellino corneal dystrophy are heterozygous mutants that express both normal and mutant proteins, while patients with Avellino corneal dystrophy by homozygous mutants that express only mutant proteins are very rare, at around 1/1,000 or 1/10,000. Thus, in order to develop a therapeutic agent, it is necessary to inhibit the overall TGFBI, but an additional function is required to minimize the effect on wild-type TGFBI and selectively inhibit the R124H mutant TGFBI.

Accordingly, the inventors of the present disclosure conceived that preventing the production of the R124H mutant TGFBI protein would be a highly effective therapeutic strategy for treating Avellino corneal dystrophy, and based on this insight, completed the present disclosure by identifying a novel ASO nucleotide sequence that inhibits the expression of the R124H TGFBI protein.

In addition, since normal WT TGFBI protein plays a role in cell adhesion and differentiation for the maintenance of tissues in vivo, it is desirable to maintain normal WT TGFBI expression even when the expression of mutant TGFBI is inhibited. Therefore, in the present disclosure, an ASO nucleotide sequence that selectively inhibits R124H TGFBI expression while minimizing the effect on WT TGFBI expression was additionally identified.

Accordingly, the present disclosure provides a pharmaceutical composition for preventing or treating Avellino corneal dystrophy, including a single-stranded antisense oligonucleotide having a nucleotide sequence that inhibits expression of transforming growth factor β-induced (TGFBI) mRNA having an R124H mutation.

Since Avellino corneal dystrophy is caused by a point mutation of CGC, the mRNA codon for arginine (R) at position 124 of the normal TGFBI protein, to CAC, the present disclosure may include an antisense oligonucleotide having a nucleotide sequence shown in Tables 1 to 4 that directly inhibits mRNA so as to prevent protein translation by the R124H TGFBI mRNA.

Specifically, the present disclosure, when including an 18-mer antisense oligonucleotide, may be one or more types selected from among the 9 nucleotide sequences (SEQ ID NOs: 1 to 9), which comprise modified deoxyribonucleic acid (DNA), linked by phosphorothioate (PS) bonds, and the 9 nucleotide sequences (SEQ ID NOs: 10 to 18) of unmodified DNA linked by phosphorothioate (PS) bonds as shown in Table 1.

When a 19-mer antisense oligonucleotide is included, it may be one or more types selected from 10 nucleotide sequences (SEQ ID NOs: 19 to 28), which comprise modified DNA, linked by PS bonds, and the 10 nucleotide sequences (SEQ ID NOs: 29 to 38) of unmodified DNA linked by PS bonds as shown in Table 2.

When a 20-mer antisense oligonucleotide is included, it may be one or more types selected from 11 nucleotide sequences (SEQ ID NOs: 39 to 49) including modified DNA, linked by PS bonds, and the 11 nucleotide sequences (SEQ ID NOs: 50 to 60) of unmodified DNA linked by PS bonds as shown in Table 3.

In addition, the present disclosure may be one or more types selected from the nucleotide sequences (SEQ ID NOs: 61 to 67) that give one or more mismatches in addition to the R124H TGFBI complementary sequence as shown in Table 4.

The ASO sequence is basically based on deoxyribonucleic acid (DNA), and when modifications are applied, they are expressed as follows.
* = 2'-O-Methoxyethyl (2'-MOE)
m = 5-methyl
- = phosphorothioate linkage (PS linkage)

**TABLE 1**

| 18-mer antisense oligonucleotide sequence | |
|---|---|
| Sequence number (SEQ ID NO.) | Sequence (5'→ 3') |
| 1 | *mC-*mC-*G-*T-*G-T-G-G-T-mC-mC-G-T-*G-*T-*A-*mC-*A |
| 2 | *T-*mC-*mC-*G-*T-G-T-G-G-T-mC-mC-G-*T-*G-*T-*A-*mC |
| 3 | *mC-*T-*mC-*mC-*G-T-G-T-G-G-T-mC-mC-*G-*T-*G-*T-*A |
| 4 | *T-*mC-*T-*mC-*mC-G-T-G-T-G-G-T-mC-*mC-*G-*T-*G-*T |
| 5 | *T-*T-*mC-*T-*mC-mC-G-T-G-T-G-G-T-*mC-*mC-*G-*T-*G |
| 6 | *mC-*T-*T-*mC-*T-mC-mC-G-T-G-T-G-G-*T-*mC-*mC-*G-*T |
| 7 | *G-*mC-*T-*T-*mC-T-mC-mC-G-T-G-T-G-*G-*T-*mC-*mC-*G |
| 8 | *A-*G-*mC-*T-*T-mC-T-mC-mC-G-T-G-T-*G-*G-*T-*mC-*mC |
| 9 | *mC-*A-*G-*mC-*T-T-mC-T-mC-mC-G-T-G-*T-*G-*G-*T-*mC |
| 10 | C-C-G-T-G-T-G-G-T-C-C-G-T-G-T-A-C-A |
| 11 | T-C-C-G-T-G-T-G-G-T-C-C-G-T-G-T-A-C |
| 12 | C-T-C-C-G-T-G-T-G-G-T-C-C-G-T-G-T-A |
| 13 | T-C-T-C-C-G-T-G-T-G-G-T-C-C-G-T-G-T |
| 14 | T-T-C-T-C-C-G-T-G-T-G-G-T-C-C-G-T-G |
| 15 | C-T-T-C-T-C-C-G-T-G-T-G-G-T-C-C-G-T |
| 16 | G-C-T-T-C-T-C-C-G-T-G-T-G-G-T-C-C-G |
| 17 | A-G-C-T-T-C-T-C-C-G-T-G-T-G-G-T-C-C |
| 18 | C-A-G-C-T-T-C-T-C-C-G-T-G-T-G-G-T-C |

**TABLE 2**

| 19-mer antisense oligonucleotide sequence | |
|---|---|
| Sequence number (SEQ ID NO.) | Sequence (5'→ 3') |
| 19 | *mC-*mC-*G-*T-*G-T-G-G-T-mC-mC-G-T-G-*T-*A-*mC-*A-*G |
| 20 | *T-*mC-*mC-*G-*T-G-T-G-G-T-mC-mC-G-T-*G-*T-*A-*mC-*A |
| 21 | *mC-*T-*mC-*mC-*G-T-G-T-G-G-T-mC-mC-G-*T-*G-*T-*A-*mC |
| 22 | *T-*mC-*T-*mC-*mC-G-T-G-T-G-G-T-mC-mC-*G-*T-*G-*T-*A |
| 23 | *T-*T-*mC-*T-*mC-mC-G-T-G-T-G-G-T-mC-*mC-*G-*T-*G-*T |
| 24 | *mC-*T-*T-*mC-*T-mC-mC-G-T-G-T-G-G-T-*mC-*mC-*G-*T-*G |
| 25 | *G-*mC-*T-*T-*mC-T-mC-mC-G-T-G-T-G-G-*T-*mC-*mC-*G-*T |
| 26 | *A-*G-*mC-*T-*T-mC-T-mC-mC-G-T-G-T-G-*G-*T-*mC-*mC-*G |
| 27 | *mC-*A-*G-*mC-*T-T-mC-T-mC-mC-G-T-G-T-*G-*G-*T-*mC-*mC |
| 28 | *T-*mC-*A-*G-*mC-T-T-mC-T-mC-mC-G-T-G-*T-*G-*G-*T-*mC |
| 29 | C-C-G-T-G-T-G-G-T-C-C-G-T-G-T-A-C-A-G |
| 30 | T-C-C-G-T-G-T-G-G-T-C-C-G-T-G-T-A-C-A |
| 31 | C-T-C-C-G-T-G-T-G-G-T-C-C-G-T-G-T-A-C |
| 32 | T-C-T-C-C-G-T-G-T-G-G-T-C-C-G-T-G-T-A |
| 33 | T-T-C-T-C-C-G-T-G-T-G-G-T-C-C-G-T-G-T |
| 34 | C-T-T-C-T-C-C-G-T-G-T-G-G-T-C-C-G-T-G |
| 35 | G-C-T-T-C-T-C-C-G-T-G-T-G-G-T-C-C-G-T |
| 36 | A-G-C-T-T-C-T-C-C-G-T-G-T-G-G-T-C-C-G |
| 37 | C-A-G-C-T-T-C-T-C-C-G-T-G-T-G-G-T-C-C |
| 38 | T-C-A-G-C-T-T-C-T-C-C-G-T-G-T-G-G-T-C |

**TABLE 3**

| 20-mer antisense oligonucleotide sequence | |
|---|---|
| Sequence number (SEQ ID NO.) | Sequence (5'→ 3') |
| 39 | *mC-*mC-*G-*T-*G-T-G-G-T-mC-mC-G-T-G-T-*A-*mC-*A-*G- *mC |
| 40 | *T-*mC-*mC-*G-*T-G-T-G-G-T-mC-mC-G-T-G-*T-*A-*mC-*A-*G |
| 41 | *mC-*T-*mC-*mC-*G-T-G-T-G-G-T-mC-mC-G-T-*G-*T-*A-*mC- *A |
| 42 | *T-*mC-*T-*mC-*mC-G-T-G-T-G-G-T-mC-mC-G-*T-*G-*T-*A- *mC |
| 43 | *T-*T-*mC-*T-*mC-mC-G-T-G-T-G-G-T-mC-mC-*G-*T-*G-*T-*A |
| 44 | *mC-*T-*T-*mC-*T-mC-mC-G-T-G-T-G-G-T-mC-*mC-*G-*T-*G- *T |
| 45 | *G-*mC-*T-*T-*mC-T-mC-mC-G-T-G-T-G-G-T-*mC-*mC-*G-*T- *G |
| 46 | *A-*G-*mC-*T-*T-mC-T-mC-mC-G-T-G-T-G-G-*T-*mC-*mC-*G- *T |
| 47 | *mC-*A-*G-*mC-*T-T-mC-T-mC-mC-G-T-G-T-G-*G-*T-*mC- *mC-*G |
| 48 | *T-*mC-*A-*G-*mC-T-T-mC-T-mC-mC-G-T-G-T-*G-*G-*T-*mC- *mC |
| 49 | *mC-*T-*mC-*A-*G-mC-T-T-mC-T-mC-mC-G-T-G-*T-*G-*G-*T- *mC |
| 50 | C-C-G-T-G-T-G-G-T-C-C-G-T-G-T-A-C-A-G-C |
| 51 | T-C-C-G-T-G-T-G-G-T-C-C-G-T-G-T-A-C-A-G |
| 52 | C-T-C-C-G-T-G-T-G-G-T-C-C-G-T-G-T-A-C-A |
| 53 | T-C-T-C-C-G-T-G-T-G-G-T-C-C-G-T-G-T-A-C |
| 54 | T-T-C-T-C-C-G-T-G-T-G-G-T-C-C-G-T-G-T-A |
| 55 | C-T-T-C-T-C-C-G-T-G-T-G-G-T-C-C-G-T-G-T |
| 56 | G-C-T-T-C-T-C-C-G-T-G-T-G-G-T-C-C-G-T-G |
| 57 | A-G-C-T-T-C-T-C-C-G-T-G-T-G-G-T-C-C-G-T |
| 58 | C-A-G-C-T-T-C-T-C-C-G-T-G-T-G-G-T-C-C-G |
| 59 | T-C-A-G-C-T-T-C-T-C-C-G-T-G-T-G-G-T-C-C |
| 60 | C-T-C-A-G-C-T-T-C-T-C-C-G-T-G-T-G-G-T-C |

**TABLE 4**

| Mismatch sequence modified ASO sequence | |
|---|---|
| Sequence number (SEQ ID NO.) | Sequence (5'→ 3') |
| 61 | T-T-C-T-C-G-G-T-G-T-G-G-T-C-C-G-T-G |
| 62 | T-T-C-T-C-C-C-T-G-T-G-G-T-C-C-G-T-G |
| 63 | T-T-C-T-C-C-G-A-G-T-G-G-T-C-C-G-T-G |
| 64 | T-T-C-T-C-C-G-T-C-T-G-G-T-C-C-G-T-G |
| 65 | T-T-C-T-C-C-G-T-G-T-C-G-T-C-C-G-T-G |
| 66 | T-T-C-T-C-C-G-T-G-T-G-C-T-C-C-G-T-G |
| 67 | T-T-C-T-C-C-G-T-G-T-G-G-A-C-C-G-T-G |

In particular, the present disclosure provides a pharmaceutical composition for preventing or treating Avellino corneal dystrophy, including one or more types of antisense oligonucleotide selected from the group consisting of SEQ ID NOs: 1, 2, 3, 4, 5, 6, 7, 8, 14, 17, 22, 23, 24, 43, 44, and 45 among the nucleotide sequences of the following Table 1, which inhibit R124H TGFBI expression.

Preferably, the present disclosure provides a pharmaceutical composition for preventing or treating Avellino corneal dystrophy, including one or more types of antisense oligonucleotide selected from the group consisting of SEQ ID NOs: 2, 3, 4, 5, 6, 7, 8, 22, 23, 24, 43, 44, and 45 as a nucleotide sequence that selectively inhibits R124H TGFBI expression while minimizing the effect on WT TGFBI expression, and more preferably, one or more types of antisense oligonucleotide selected from the group consisting of SEQ ID NOs: 2, 3, 4, 5, 6, 7, 8, 22, 23, and 45.

On the one hand, the present disclosure provides a use as an agent for preventing or treating Avellino corneal dystrophy, including one or more types of antisense oligonucleotide selected from the group consisting of SEQ ID NOs: 1, 2, 3, 4, 5, 6, 7, 8, 14, 17, 22, 23, 24, 43, 44, and 45 as a nucleotide sequence that inhibits expression of R124H TGFBI mRNA.

Preferably, the present disclosure provides a use as an agent for preventing or treating Avellino corneal dystrophy, including one or more types of antisense oligonucleotide selected from the group consisting of SEQ ID NOs: 2, 3, 4, 5, 6, 7, 8, 22, 23, 24, 43, 44, and 45 as a nucleotide sequence that selectively inhibits R124H TGFBI expression while minimizing the effect on WT TGFBI expression, and more preferably, one or more types of antisense oligonucleotide selected from the group consisting of SEQ ID NOs: 2, 3, 4, 5, 6, 7, 8, 22, 23, and 45.

On the other hand, the present disclosure provides a method of preventing or treating Avellino corneal dystrophy, including administering an antisense oligonucleotide having a nucleotide sequence that inhibits R124H TGFBI expression. In particular, the present disclosure provides a method of preventing or treating Avellino corneal dystrophy, wherein the nucleotide sequence includes one or more types of antisense oligonucleotide selected from the group consisting of SEQ ID NOs: 1, 2, 3, 4, 5, 6, 7, 8, 14, 17, 22, 23, 24, 43, 44, and 45.

Preferably, the present disclosure provides a method of preventing or treating Avellino corneal dystrophy, including one or more types of antisense oligonucleotide selected from the group consisting of SEQ ID NOs: 2, 3, 4, 5, 6, 7, 8, 22, 23, 24, 43, 44, and 45 as a nucleotide sequence that selectively inhibits R124H TGFBI expression while minimizing the effect on WT TGFBI expression, and more preferably, one or more types of antisense oligonucleotide selected from the group consisting of SEQ ID NOs: 2, 3, 4, 5, 6, 7, 8, 22, 23, and 45.

In addition, although the present disclosure is structurally based on 18-mer to 20-mer, it does not depart from the scope of the present disclosure even if the nucleotide sequences of SEQ ID NOs: 1 to 67 are used and 1 to 6 nucleic acids or nucleic acid derivatives are additionally formed at the 5' and 3' end thereof. The present disclosure does not depart from the scope of the present disclosure even if the nucleotide sequences of SEQ ID NOs: 1 to 67 are used and additionally a biodegradable or non-biodegradable linker is linked to the 5' or 3' end thereof or another compound is additionally formed without linkage. The present disclosure encompasses the use of the nucleotide sequences of SEQ ID NOs: 1 to 67, and further includes embodiments in which biodegradable or non-biodegradable linkers are attached either to a sugar moiety or a nucleobase of an internal nucleotide, or to an internucleotide linkage or in which other compounds are formed without such linkers, without departing from the scope of the present disclosure.

Additionally, the present disclosure provides a pharmaceutical composition for preventing or treating Avellino corneal dystrophy, wherein the antisense oligonucleotide includes a chemical modification in a sugar moiety or a nucleobase of one or more ribonucleic acids, or an internucleotide linkage of the ribonucleic acids.

That is, the antisense oligonucleotide according to the present disclosure may involve various modifications that may be conceived by those skilled in the art, but as long as the nucleotide sequence itself does not change, such modifications also fall within the scope of the present disclosure. Here, the degree to which the nucleotide sequence itself remains unchanged means that, even when a portion of the 5'- or 3'-end of the nucleotide sequence, or an internal portion thereof, is added, substituted, or deleted, approximately 90% or more of the nucleotide sequence is retained.

As an example of modification, even if the binding site of the nucleic acid has modification such as phosphorothioate, thiophosphoramidate, phosphonate, or methylphosphonate, it does not deviate from the scope of the present disclosure as long as the nucleotide sequence is not changed. In addition, even if a chemical modification such as 2'-O-methoxyethyl (2'-MOE), 2'-fluoro, 2'-O-methyl (2'-OMe), locked nucleic acid (LNA), peptide nucleic acid (PNA), phosphoramidate morpholino oligomer (PMO), N3'-P5' phosphoramidates, 2'-deoxy-2'-fluoro-β-D-arabino nucleic acid analogue (FANA), cyclohexene nucleic acids (CeNAs), tricyclo-DNA (tcDNA), 2-thiothymidine, 3'-fluorohexitol nucleic acid (FHNA), ethylene-bridged nucleic acid (ENA), or (S)-constrained ethyl (cEt) modification is introduced into the nucleic acid structure, it does not deviate from the scope of the present disclosure as long as the nucleotide sequence remains unchanged. 5-methyl pyrimidine may be applied to a base part as needed, and even if additional fluoro is modified, it does not depart from the scope of the present disclosure.

The pharmaceutical composition according to the present disclosure may be provided in one or more formulations selected from the group consisting of gels, emulsions, injections, and aerosols according to a conventional method, but is not limited thereto.

In another embodiment of the present disclosure, the pharmaceutical composition may further include one or more additives selected from the group consisting of suitable carriers and diluents, antioxidants, buffers, bacteriostatic agents, dispersing agents, and surfactants that are commonly used in the manufacture of pharmaceutical compositions.

Specifically, lactose, dextrose, sucrose, sorbitol, mannitol, xylitol, erythritol, maltitol, polyvinyl pyrrolidone, and water may be used as the carrier and diluent, and in addition, non-aqueous solvents may include propylene glycol, polyethylene glycol, and vegetable oils such as olive oil, ethyl oleate, and synthetic oils.

The above pharmaceutical composition may be administered to a subject in a conventional manner via intravenous, intraarterial, intraperitoneal, intramuscular, intrasternal, transdermal, intranasal, inhalation, topical, rectal, intraocular, or intradermal routes.

The preferred dosage of the antisense oligonucleotide may vary depending on the condition and weight of a subject, the type and extent of the disease, the drug form, the route, and period of administration and may be appropriately selected by those skilled in the art, and the scope of the present disclosure is not limited thereby.

In the present disclosure, the 'subject' may be a mammal including human, but is not limited to these examples.

Hereinafter, the present disclosure will be described in more detail through examples. These examples are only intended to more specifically illustrate the present disclosure, and it will be apparent to those skilled in the art to which the present disclosure pertains that the scope of the present disclosure is not limited by these examples according to the gist of the present disclosure.

### Examples 1-67: Preparation of antisense oligonucleotides targeting R124H TGFBI mRNA

Antisense oligonucleotides for respective SEQ ID NOs were prepared using an oligonucleotide synthesizer (Oligo synthesizer). Synthesis was carried out by applying modifications based on DNA according to the sequence notation in Table 5.

### <Applied modification>

* = 2'-O-Methoxyethyl (2'-MOE)
m = 5-methyl
- = phosphorothioate linkage (PS linkage)

**TABLE 5**

| Example | Sequence (5'→ 3') | Sequence number (SEQ ID NO.) |
|---|---|---|
| Example 1 | *mC-*mC-*G-*T-*G-T-G-G-T-mC-mC-G-T-*G-*T-*A-*mC- *A | 1 |
| Example 2 | *T-*mC-*mC-*G-*T-G-T-G-G-T-mC-mC-G-*T-*G-*T-*A- *mC | 2 |
| Example | *mC-*T-*mC-*mC-*G-T-G-T-G-G-T-mC-mC-*G-*T-*G-*T- | 3 |
| 3 | *A | |
| Example 4 | *T-*mC-*T-*mC-*mC-G-T-G-T-G-G-T-mC-*mC-*G-*T-*G- *T | 4 |
| Example 5 | *T-*T-*mC-*T-*mC-mC-G-T-G-T-G-G-T-*mC-*mC-*G-*T- *G | 5 |
| Example 6 | *mC-*T-*T-*mC-*T-mC-mC-G-T-G-T-G-G-*T-*mC-*mC- *G-*T | 6 |
| Example 7 | *G-*mC-*T-*T-*mC-T-mC-mC-G-T-G-T-G-*G-*T-*mC- *mC-*G | 7 |
| Example 8 | *A-*G-*mC-*T-*T-mC-T-mC-mC-G-T-G-T-*G-*G-*T-*mC- *mC | 8 |
| Example 9 | *mC-*A-*G-*mC-*T-T-mC-T-mC-mC-G-T-G-*T-*G-*G-*T- *mC | 9 |
| Example 10 | C-C-G-T-G-T-G-G-T-C-C-G-T-G-T-A-C-A | 10 |
| Example 11 | T-C-C-G-T-G-T-G-G-T-C-C-G-T-G-T-A-C | 11 |
| Example 12 | C-T-C-C-G-T-G-T-G-G-T-C-C-G-T-G-T-A | 12 |
| Example 13 | T-C-T-C-C-G-T-G-T-G-G-T-C-C-G-T-G-T | 13 |
| Example 14 | T-T-C-T-C-C-G-T-G-T-G-G-T-C-C-G-T-G | 14 |
| Example 15 | C-T-T-C-T-C-C-G-T-G-T-G-G-T-C-C-G-T | 15 |
| Example 16 | G-C-T-T-C-T-C-C-G-T-G-T-G-G-T-C-C-G | 16 |
| Example 17 | A-G-C-T-T-C-T-C-C-G-T-G-T-G-G-T-C-C | 17 |
| Example 18 | C-A-G-C-T-T-C-T-C-C-G-T-G-T-G-G-T-C | 18 |
| Example 19 | *mC-*mC-*G-*T-*G-T-G-G-T-mC-mC-G-T-G-*T-*A-*mC- *A-*G | 19 |
| Example 20 | *T-*mC-*mC-*G-*T-G-T-G-G-T-mC-mC-G-T-*G-*T-*A- *mC-*A | 20 |
| Example 21 | *mC-*T-*mC-*mC-*G-T-G-T-G-G-T-mC-mC-G-*T-*G-*T- *A-*mC | 21 |
| Example 22 | *T-*mC-*T-*mC-*mC-G-T-G-T-G-G-T-mC-mC-*G-*T-*G- *T-*A | 22 |
| Example 23 | *T-*T-*mC-*T-*mC-mC-G-T-G-T-G-G-T-mC-*mC-*G-*T- *G-*T | 23 |
| Example 24 | *mC-*T-*T-*mC-*T-mC-mC-G-T-G-T-G-G-T-*mC-*mC- *G-*T-*G | 24 |
| Example 25 | *G-*mC-*T-*T-*mC-T-mC-mC-G-T-G-T-G-G-*T-*mC- *mC-*G-*T | 25 |
| Example 26 | *A-*G-*mC-*T-*T-mC-T-mC-mC-G-T-G-T-G-*G-*T-*mC- *mC-*G | 26 |
| Example 27 | *mC-*A-*G-*mC-*T-T-mC-T-mC-mC-G-T-G-T-*G-*G-*T- *mC-*mC | 27 |
| Example 28 | *T-*mC-*A-*G-*mC-T-T-mC-T-mC-mC-G-T-G-*T-*G-*G- *T-*mC | 28 |
| Example 29 | C-C-G-T-G-T-G-G-T-C-C-G-T-G-T-A-C-A-G | 29 |
| Example 30 | T-C-C-G-T-G-T-G-G-T-C-C-G-T-G-T-A-C-A | 30 |
| Example 31 | C-T-C-C-G-T-G-T-G-G-T-C-C-G-T-G-T-A-C | 31 |
| Example 32 | T-C-T-C-C-G-T-G-T-G-G-T-C-C-G-T-G-T-A | 32 |
| Example 33 | T-T-C-T-C-C-G-T-G-T-G-G-T-C-C-G-T-G-T | 33 |
| Example 34 | C-T-T-C-T-C-C-G-T-G-T-G-G-T-C-C-G-T-G | 34 |
| Example 35 | G-C-T-T-C-T-C-C-G-T-G-T-G-G-T-C-C-G-T | 35 |
| Example 36 | A-G-C-T-T-C-T-C-C-G-T-G-T-G-G-T-C-C-G | 36 |
| Example 37 | C-A-G-C-T-T-C-T-C-C-G-T-G-T-G-G-T-C-C | 37 |
| Example 38 | T-C-A-G-C-T-T-C-T-C-C-G-T-G-T-G-G-T-C | 38 |
| Example 39 | *mC-*mC-*G-*T-*G-T-G-G-T-mC-mC-G-T-G-T-*A-*mC- *A-*G-*mC | 39 |
| Example 40 | *T-*mC-*mC-*G-*T-G-T-G-G-T-mC-mC-G-T-G-*T-*A- *mC-*A-*G | 40 |
| Example 41 | *mC-*T-*mC-*mC-*G-T-G-T-G-G-T-mC-mC-G-T-*G-*T- *A-*mC-*A | 41 |
| Example 42 | *T-*mC-*T-*mC-*mC-G-T-G-T-G-G-T-mC-mC-G-*T-*G- *T-*A-*mC | 42 |
| Example 43 | *T-*T-*mC-*T-*mC-mC-G-T-G-T-G-G-T-mC-mC-*G-*T- *G-*T-*A | 43 |
| Example | *mC-*T-*T-*mC-*T-mC-mC-G-T-G-T-G-G-T-mC-*mC-*G- | 44 |
| 44 | *T-*G-*T | |
| Example 45 | *G-*mC-*T-*T-*mC-T-mC-mC-G-T-G-T-G-G-T-*mC-*mC- *G-*T-*G | 45 |
| Example 46 | *A-*G-*mC-*T-*T-mC-T-mC-mC-G-T-G-T-G-G-*T-*mC- *mC-*G-*T | 46 |
| Example 47 | *mC-*A-*G-*mC-*T-T-mC-T-mC-mC-G-T-G-T-G-*G-*T- *mC-*mC-*G | 47 |
| Example 48 | *T-*mC-*A-*G-*mC-T-T-mC-T-mC-mC-G-T-G-T-*G-*G- *T-*mC-*mC | 48 |
| Example 49 | *mC-*T-*mC-*A-*G-mC-T-T-mC-T-mC-mC-G-T-G-*T-*G- *G-*T-*mC | 49 |
| Example 50 | C-C-G-T-G-T-G-G-T-C-C-G-T-G-T-A-C-A-G-C | 50 |
| Example 51 | T-C-C-G-T-G-T-G-G-T-C-C-G-T-G-T-A-C-A-G | 51 |
| Example 52 | C-T-C-C-G-T-G-T-G-G-T-C-C-G-T-G-T-A-C-A | 52 |
| Example 53 | T-C-T-C-C-G-T-G-T-G-G-T-C-C-G-T-G-T-A-C | 53 |
| Example 54 | T-T-C-T-C-C-G-T-G-T-G-G-T-C-C-G-T-G-T-A | 54 |
| Example 55 | C-T-T-C-T-C-C-G-T-G-T-G-G-T-C-C-G-T-G-T | 55 |
| Example 56 | G-C-T-T-C-T-C-C-G-T-G-T-G-G-T-C-C-G-T-G | 56 |
| Example 57 | A-G-C-T-T-C-T-C-C-G-T-G-T-G-G-T-C-C-G-T | 57 |
| Example 58 | C-A-G-C-T-T-C-T-C-C-G-T-G-T-G-G-T-C-C-G | 58 |
| Example 59 | T-C-A-G-C-T-T-C-T-C-C-G-T-G-T-G-G-T-C-C | 59 |
| Example 60 | C-T-C-A-G-C-T-T-C-T-C-C-G-T-G-T-G-G-T-C | 60 |
| Example 61 | T-T-C-T-C-G-G-T-G-T-G-G-T-C-C-G-T-G | 61 |
| Example 62 | T-T-C-T-C-C-C-T-G-T-G-G-T-C-C-G-T-G | 62 |
| Example 63 | T-T-C-T-C-C-G-A-G-T-G-G-T-C-C-G-T-G | 63 |
| Example 64 | T-T-C-T-C-C-G-T-C-T-G-G-T-C-C-G-T-G | 64 |
| Example 65 | T-T-C-T-C-C-G-T-G-T-C-G-T-C-C-G-T-G | 65 |
| Example 66 | T-T-C-T-C-C-G-T-G-T-G-C-T-C-C-G-T-G | 66 |
| Example 67 | T-T-C-T-C-C-G-T-G-T-G-G-A-C-C-G-T-G | 67 |

### Experimental Example 1: Establishment of a reporter gene system for evaluating an inhibitory ability on expression of human WT TGFBI and human R124H TGFBI

In order to derive an ASO that selectively acts on R124H TGFBI compared to WT TGFBI, a system was constructed, in which a reporter gene is also expressed when the protein is expressed for WT TGFBI and R124H TGFBI, respectively. As shown in FIG. 1, human WT TGFBI CDS or human R124H TGFBI CDS was inserted into the psiCHECK-2 vector (Promega), and Renilla luciferase, a reporter gene, was designed to be expressed upon protein expression for human WT TGFBI or human R124H TGFBI, respectively. In the vector, a control reporter, the firefly luciferase gene, which is designed to be expressed when the plasmid is transfected, was inserted to normalize the degree of transfection of the plasmid, enabling acquisition of accurate and reproducible results on the effect on expression of the target gene, WT TGFBI or R124H TGFBI.

### Experimental Example 2: Evaluation of an inhibitory ability on human WT TGFBI and human R124H TGFBI expression (evaluation through transfection of ASO)

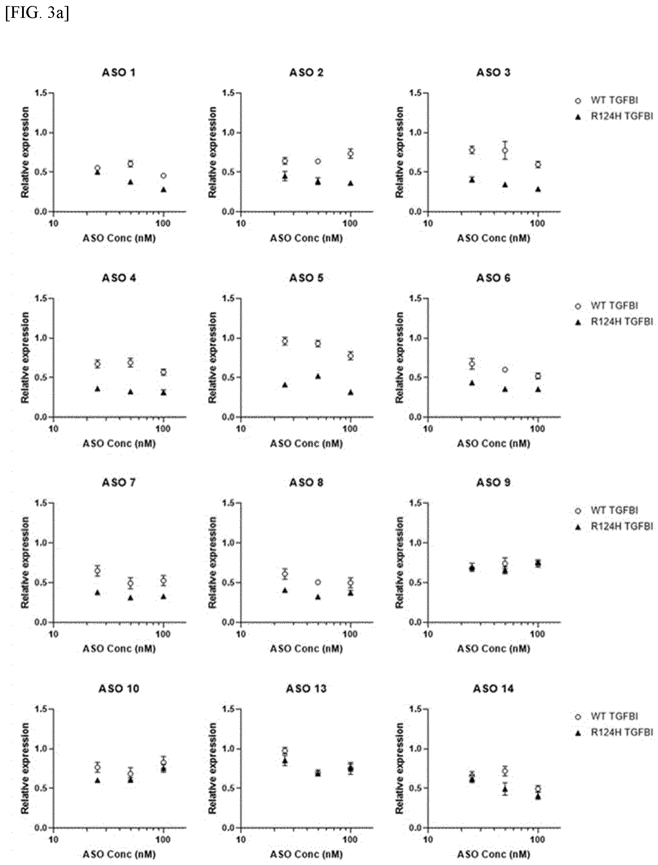

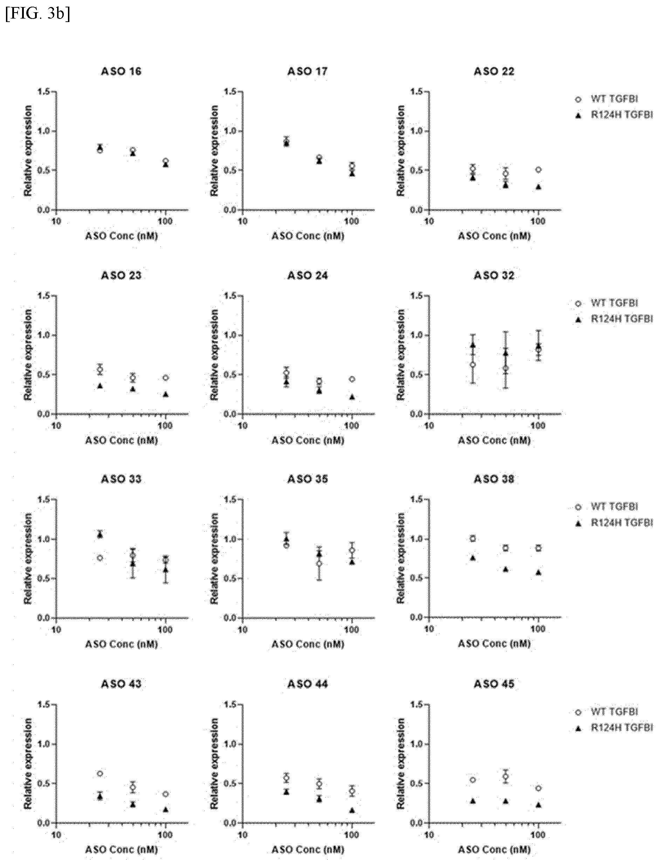

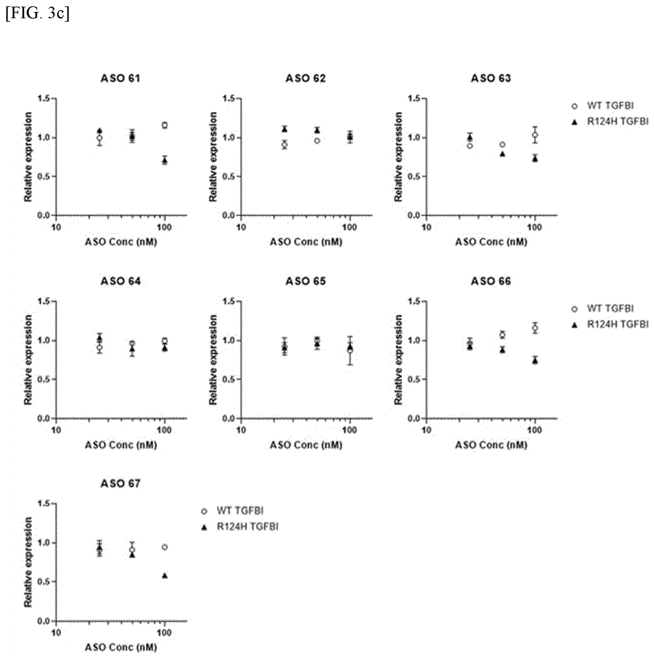

The plasmids of Experimental Example 1 (psi-CHECK2_WT TGFBI and psiCHECK2_R124H TGFBI) were individually transfected into HEK-293 cells, which exhibit very low endogenous TGFBI expression levels, to evaluate the examples. HEK-293 cells were seeded in a 96-well plate at a density of 1.7 x 10⁴cells/well and cultured overnight in DMEM (Thermo Fisher Scientific) medium containing 10% FBS at 37°C in the presence of 5% CO₂. psi-CHECK2_WT TGFBI plasmid or psi-CHECK2_R124H TGFBI plasmid was transfected into different wells of a 96-well plate by 20 ng per well using lipofectamine 2000 (Thermo Fisher Scientific), and 6 hours later, the ASOs of the Examples were transfected to make 0, 25, 50, and 100 nM per well using lipofectamine RNAiMAX (Thermo Fisher Scientific). 24 hours after ASO treatment, luminescence resulting from the expression of Renilla luciferase and firefly luciferase was measured using a plate reader (GloMax, Promega) via the Dual-Glo luciferase assay system (Promega). The luminescence value for Renilla luciferase, measured for each well, was divided by the luminescence value for firefly luciferase for correction, and then, the value for the control group not treated with ASO was set to 1 to relatively represent the value for the ASO treated group as in FIG. 3.

As a result, the ASOs that inhibited R124H human TGFBI expression by 50% or more at a concentration of 25 nM were Examples 2, 3, 4, 5, 6, 7, 8, 22, 23, 24, 43, 44, and 45, and their nucleotide sequences corresponded to SEQ ID NOs: 2, 3, 4, 5, 6, 7, 8, 22, 23, 24, 43, 44, and 45, respectively. In addition, ASOs that inhibited R124H human TGFBI expression by 50% or more at a concentration of 25 nM while failing to inhibit WT TGFBI expression by 50% or more at the same concentration were Examples 2, 3, 4, 5, 6, 7, 8, 22, 23, 24, 43, 44, and 45, and their nucleotide sequences corresponded to SEQ ID NOs: 2, 3, 4, 5, 6, 7, 8, 22, 23, 24, 43, 44, and 45, respectively. In addition, ASOs that inhibited R124H human TGFBI expression by 50% or more at a concentration of 25 nM, while failing to inhibit WT TGFBI expression by 50% or more at 50 nM, were Examples 2, 3, 4, 5, 6, 7, 8, and 45, and their nucleotide sequences corresponded to SEQ ID NOs: 2, 3, 4, 5, 6, 7, 8, and 45, respectively. In addition, ASOs that inhibited R124H human TGFBI expression by 50% or more at a concentration of 25 nM, while failing to inhibit WT TGFBI expression by 50% or more at 100 nM, were Examples 2, 3, 4, 5, 6, and 7, and their nucleotide sequences corresponded to SEQ ID NOs: 2, 3, 4, 5, 6, and 7, respectively.

In addition to the above sequence, ASOs that inhibited R124H human TGFBI expression by 50% or more at a treatment concentration of 50 nM were Examples 1 and 14, and these Examples did not inhibit WT TGFBI expression by 50% or more at the same concentration and their nucleotide sequences corresponded to SEQ ID NOs: 1 and 14, respectively.

In addition to the above sequence, an ASO that inhibited R124H human TGFBI expression by 50% or more at a treatment concentration of 100 nM was Example 17, and the Example did not inhibit WT TGFBI expression by 50% or more at the same concentration and its nucleotide sequence corresponded to SEQ ID NO: 17.

In addition, ASOs of Examples 1, 2, 3, 4, 5, 6, 7, 8, 14, 22, 23, 24, 38, 43, 44, 45, 61, 63, 66, and 67 exhibited an inhibition rate against R124H human TGFBI expression that was at least 20% higher than that against WT human TGFBI, regardless of treatment concentration, and their nucleotide sequences corresponded to SEQ ID NOs: 1, 2, 3, 4, 5, 6, 7, 8, 14, 22, 23, 24, 38, 43, 44, 45, 61, 63, 66, and 67, respectively.

In addition, ASOs of Examples 2, 3, 4, 5, 38, 45, 61, 66, and 67 exhibited an inhibition rate against R124H human TGFBI expression that was at least 30% higher than that against WT human TGFBI, regardless of treatment concentration, and their nucleotide sequences corresponded to SEQ ID NOs: 2, 3, 4, 5, 38, 45, 61, 66, and 67, respectively.

Further, as a result of experiments conducted on ASO 9, 10, 13, 16, 32, 33, 35, 38, etc., it was found that ASO 9, 10, 13, and 16 with sequence numbers of 18-mer or less, or ASO 32, 33, 35, and 38 with sequence numbers of 19-mer or more, had an inhibitory effect of less than 50% for the R124H mutant TGFBI, and there was almost no difference in the inhibitory effect on the wild-type TGFBI and the R124H mutant TGFBI, or conversely, the wild-type TGFBI was inhibited more (FIG. 3).

### Experimental Example 3: Evaluation of an inhibitory ability on human WT TGFBI and human R124H TGFBI expression (evaluation through gymnotic delivery of ASO)

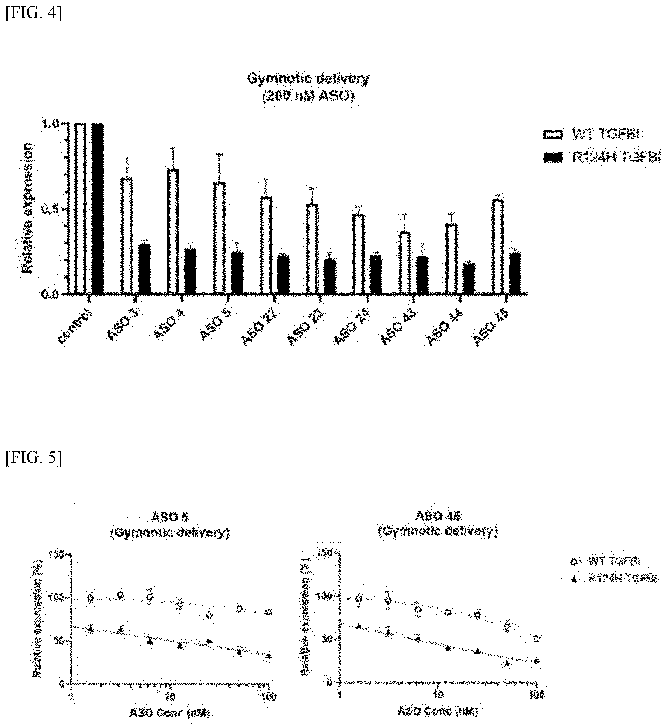

Additional evaluation was conducted on the extent of expression inhibition for WT TGFBI and R124H TGFBI through gymnotic delivery into cells by ASO treatment itself without transfection reagents or other delivery aids. HEK-293 cells were seeded into a 96-well plate at a density of 1.7x10⁴cells/well and cultured overnight in DMEM medium containing 10% FBS at 37°C in the presence of 5% CO₂. psi-CHECK2_WT TGFBI plasmid or psi-CHECK2_R124H TGFBI plasmid was transfected into different wells in the 96-well plate at 20 ng per well using lipofectamine 2000 (Thermo Fisher Scientific), and after 6 hours, the ASOs of Examples 3, 4, 5, 22, 23, 24, 43, 44, and 45 were diluted with culture medium to make 0 and 200 nM per well, respectively. 24 hours after ASO treatment, luminescence resulting from the expression of Renilla luciferase and firefly luciferase was measured using a plate reader (GloMax, Promega) using the Dual-Glo luciferase assay system (Promega). The luminescence value for Renilla luciferase measured for each well was divided by the luminescence value for firefly luciferase for correction, the value for the control group not treated with ASO was then set to 1, and the value for the ASO treated group was expressed relatively as in FIG. 4.

As a result, ASOs that inhibited R124H human TGFBI expression by 50% or more were Examples 3, 4, 5, 22, 23, 24, 43, 44, and 45, each nucleotide sequence of which included the sequence of SEQ ID NOs: 3, 4, 5, 22, 23, 24, 43, 44, and 45. In addition, ASOs that inhibited R124H human TGFBI expression by 50% or more while failing to inhibit WT TGFBI expression by 50% or more were Examples 3, 4, 5, 22, 23, and 45, each nucleotide sequence of which included the sequence of SEQ ID NOs: 3, 4, 5, 22, 23, and 45.

In addition, ASOs whose expression inhibition rate of R124H human TGFBI was 20% or higher than that of WT human TGFBI were Examples 3, 4, 5, 22, 23, 24, 44, and 45, the nucleotide sequences of which included sequences of SEQ ID NOs: 3, 4, 5, 22, 23, 24, 44, and 45.

In addition, ASOs whose expression inhibition rate of R124H human TGFBI was 30% or higher than that of WT human TGFBI were Examples 3, 4, 5, 22, 23, and 45, the nucleotide sequences of which included sequences of SEQ ID NOs: 3, 4, 5, 22, 23, and 45.

Additionally, for the same experimental procedure, as an example, Examples 5 and 45 were treated at various concentrations from 0.78 nM to 100 nM, and IC₅₀ that inhibited the expression of WT TGFBI or R124H TGFBI by 50% was derived using GraphPad Prism 9.5.1.

As a result, as shown in FIG. 5, both Examples 5 and 45 showed higher expression inhibition rates for R124H TGFBI than for WT TGFBI throughout the treatment concentration range. With these results, in the derivation of IC₅₀, the IC₅₀ for R124H TGFBI in Example 5 was derived as 10.56 nM, and the IC₅₀ for WT TGFBI as 5.71 nM. The IC₅₀ for R124H TGFBI in Example 45 was derived as 5.71 nM, and the IC₅₀ for WT TGFBI as 109.3 nM. When the selectivity for R124H TGFBI over WT TGFBI was expressed as the value (WT/Mut ratio) obtained by dividing the IC₅₀ for WT TGFBI by the IC₅₀ for R124H TGFBI, Example 5 exhibited an excellent selectivity with a WT/Mut ratio of about 91.7 and Example 45 of about 19.1.

**TABLE 6**

| IC₅₀ and R124H TGFBI selectivity (WT/Mut ratio) | | | |
|---|---|---|---|
| ASO | IC₅₀ (nM) | | WT/Mut ratio |
| | WT TGFBI | R124H TGFBI | |
| Example 5 | 967.9 | 10.56 | 91.7 |
| Example 45 | 109.3 | 5.71 | 19.1 |

In a comprehensive analysis of the above test results, it was analyzed that one or more types of antisense oligonucleotide selected from the group consisting of SEQ ID NOs: 1, 2, 3, 4, 5, 6, 7, 8, 14, 17, 22, 23, 24, 43, 44, and 45 as an ASO that inhibits R124H human TGFBI expression by 50% or more at the total concentration tested in Experimental Example 2 may be useful to inhibit R124H TGFBI expression.

Preferably, it was analyzed that one or more types of antisense oligonucleotide selected from the group consisting of SEQ ID NOs: 2, 3, 4, 5, 6, 7, 8, 22, 23, 24, 43, 44, and 45 as an ASO that inhibits R124H human TGFBI expression by 50% or more at the lowest concentration of 25 nM in Experimental Example 2 and does not inhibit WT TGFBI expression by 50% or more at the same concentration may be useful to selectively inhibit R124H TGFBI expression while minimizing the effect on WT TGFBI expression.

More preferably, it was analyzed, as the union of the group consisting of SEQ ID NOs: 2, 3, 4, 5, 6, 7, 8, and 45 as ASOs that inhibit R124H human TGFBI expression by 50% or more at the lowest concentration of 25 nM in Experimental Example 2 and fail to inhibit WT TGFBI expression by 50% or more at the upper concentration of 50 nM and the group consisting of SEQ ID NOs: 3, 4, 5, 22, 23, and 45 as ASOs that inhibit R124H human TGFBI expression by 50% or more through gymnotic delivery (200 nM) without a delivery in Experimental Example 3 and fail to inhibit WT TGFBI expression by 50% or more, that one or more types of antisense oligonucleotide selected from the group consisting of SEQ ID NOs: 2, 3, 4, 5, 6, 7, 8, 22, 23, and 45 may be useful to selectively inhibit R124H TGFBI expression while minimizing the effect on WT TGFBI expression.

While specific parts of the present disclosure have been described in detail above, it is clear to those skilled in the art that such detailed descriptions ar merely preferred example embodiments, and the scope of the present disclosure is not limited thereby. In other words, the substantial scope of the present disclosure is defined by the appended claims and their equivalents.

## Claims

1. A pharmaceutical composition for preventing or treating Avellino corneal dystrophy, comprising a single-stranded antisense oligonucleotide having a nucleotide sequence that inhibits the expression of transforming growth factor β-induced (TGFBI) having an R124H mutation, wherein the antisense oligonucleotide is selected from one or more oligonucleotides from the group consisting of SEQ ID NOs: 1, 2, 3, 4, 5, 6, 7, 8, 14, 17, 22, 23, 24, 43, 44, and 45.

2. The pharmaceutical composition of claim 1, wherein the antisense oligonucleotide comprises one or more oligonucleotides selected from the group consisting of SEQ ID NOs: 2, 3, 4, 5, 6, 7, 8, 22, 23, 24, 43, 44, and 45.

3. The pharmaceutical composition of claim 1, wherein the antisense oligonucleotide comprises one or more oligonucleotides selected from the group consisting of SEQ ID NOs: 2, 3, 4, 5, 6, 7, 8, 22, 23, and 45.

4. The pharmaceutical composition of claim 1, wherein the antisense oligonucleotide comprises one or more chemical modifications in the sugar moiety, nucleobase structure, or internucleotide linkage of the ribonucleic acids.
